# EUROPEAN PATENT APPLICATION

(11) **EP 4 817 524 A1**
(43) Date of publication of application: **07.10.2026**
(21) Application number: 25167461.0
(22) Date of filing: 31.03.2025
(51) Int. Cl.: A61B 6/00

(54) **SYSTEM FOR PLANNING AN INTRAVASCULAR PROCEDURE**

(71) Applicant: Koninklijke Philips N.V., 5656 AE Eindhoven (NL)
(72) Inventor: HAASE, Hans Christian, Eindhoven (NL); CONTINI, Enrico, Eindhoven (NL)
(74) Representative: Philips Intellectual Property & Standards

(57) **Abstract**

A system (100) for planning an intravascular procedure, comprises an X-ray imaging system (110), and one or more processors (120). The X-ray imaging system (110) is configured to: acquire (S110) volumetric X-ray data (130) representing a distribution of a contrast agent injection in a region of interest in the brain; and acquire (S120) projection X-ray data (140) representing a distribution of the same contrast agent injection in the aortic arch. The one or more processors (120) are configured to: generate (S130), from the volumetric X-ray data (130), a first image (150) for assessing the region of interest in the brain; and generate (S140), from the projection X-ray data (140), a second image (160) for planning an intravascular procedure via the aortic arch.

## Description

### TECHNICAL FIELD

The present disclosure relates to planning an intravascular procedure. A system, a computer-implemented method, and a computer program product, are disclosed.

### BACKGROUND

Investigations for suspected stroke typically begin with the acquisition of a volumetric angiographic image of the brain. This image is acquired subsequent to a contrast agent injection and enables a physician to investigate the presence, and also severity, of a suspected occlusion. If an intravascular treatment procedure is deemed necessary, the physician may then navigate various intravascular devices to the brain, via the aortic arch, in order to perform the necessary treatment procedure. The navigation of such devices is typically performed using X-ray imaging. Examples of such intravascular treatment procedures include mechanical thrombectomy procedures to remove a blood clot from the blood vessel, mechanical thrombolysis procedures to break-up a clot, angioplasty, and stenting. It is important that such treatment procedures are carried out as soon as possible because the amount of long term damage to the brain increases whilst the vessel remains occluded.

However, the navigation of such intravascular devices past the aortic arch under X-ray imaging is highly complex. This is due to the tortuous shape of the aortic arch, and also due to its poor visibility in the absence of contrast agent. Sometimes a pre-procedural image of the aortic arch is available. The pre-procedural image may be registered to X-ray images of the interventional device that are acquired during navigation in order to support the navigation of the interventional device past the aortic arch. In the absence of a pre-procedural image of the aortic arch, one option is to record an angiogram of the aortic arch during navigation. However, the recording of such an angiogram is complicated by the need for an additional contrast agent injection. This contrast agent injection is typically large due to the high blood flow in the aortic arch, and it typically requires the use of a special pigtail catheter. The absence of a pre-procedural image of the aortic arch can therefore lead to additional complexity, and also complications, that delay the treatment of an occluded vessel.

Thus, there is a need to support the planning of intravascular procedures that are performed in the brain, via the aortic arch.

### SUMMARY

The invention is defined by the claims.

According to one aspect of the present disclosure, a system for planning an intravascular procedure, is provided. The system includes an X-ray imaging system and one or more processors. The X-ray imaging system is configured to acquire volumetric X-ray data representing a distribution of a contrast agent injection in a region of interest in the brain. The X-ray imaging system is also configured to acquire projection X-ray data representing a distribution of the same contrast agent injection in the aortic arch. The one or more processors are configured to generate, from the volumetric X-ray data, a first image for assessing the region of interest in the brain. The one or more processors are also configured to generate, from the projection X-ray data, a second image for planning an intravascular procedure via the aortic arch.

The above-described system therefore provides a first image for assessing the region of interest in the brain, and also a second image for planning an intravascular procedure via the aortic arch. Since the images represent distributions of the same contrast agent injection into a subject, the system provides the second image for planning the intravascular procedure via the aortic arch, without the need for an additional contrast agent injection. This avoids the unnecessary complications, and also the unnecessary contrast agent dose, associated with an additional contrast agent injection. Consequently, it may help to avoid unnecessary delays in performing the intravascular procedure.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for planning an intravascular procedure, in accordance with some aspects of the present disclosure.
Fig. 2 is a flowchart illustrating an example of a computer-implemented method of planning an intravascular procedure, in accordance with some aspects of the present disclosure.
Fig. 3 is a schematic diagram illustrating the acquisition of volumetric X-ray data 130 by a first X-ray source-detector arrangement 110a from a rotational angle θ with respect to a region of interest in the brain, and the acquisition of projection X-ray data 140 by a second X-ray source-detector arrangement 110b from an orientation α with respect to the aortic arch, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, or in a computer program product, in a corresponding manner.

In the following description, reference is made to examples of a system for planning an intravascular procedure that is performed in the brain, via the aortic arch. In some examples, reference is made to the system being used to plan an intravascular thrombectomy procedure for the treatment of a suspected occlusion in the brain, via the aortic arch. It is, however, to be appreciated that the system may be used to plan other types of intravascular procedures that are likewise performed in the brain, via the aortic arch. For instance, the system may be used to plan intravascular procedures such as thrombolysis, angioplasty, stenting, coiling, and so forth, and which are likewise performed in the brain, via the aortic arch.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the operations that are performed in the method may be performed by a single dedicated processor, or by a single shared processor, or by a plurality of individual processors, some of which can be shared. The operations that are performed in the method may for instance be performed by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a need to support the planning of intravascular procedures that are performed in the brain, via the aortic arch.

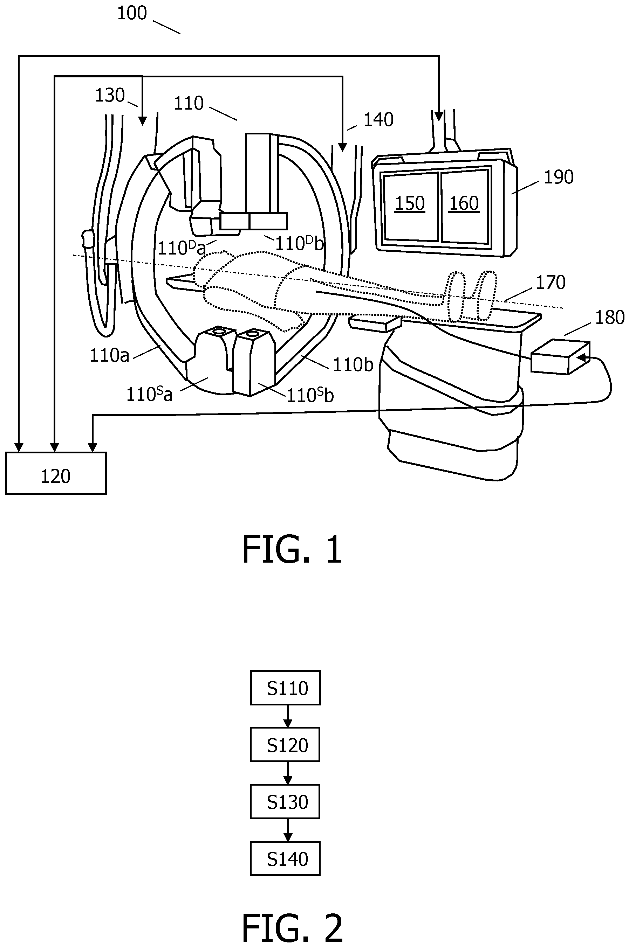

Fig. 1 is a schematic diagram illustrating an example of a system 100 for planning an intravascular procedure, in accordance with some aspects of the present disclosure. Fig. 2 is a flowchart illustrating an example of a computer-implemented method of planning an intravascular procedure, in accordance with some aspects of the present disclosure. It is noted that operations that are described as being performed by the one or more processors 120 of the system 100 illustrated in Fig. 1, may also be performed in the method illustrated in Fig. 2. Likewise, operations that are described in relation to the method described with reference to Fig. 2 may also be performed by the one or more processors 120 of the system 100 illustrated in Fig. 1. With reference to Fig. 1, and Fig. 2, the system 100 for planning an intravascular procedure, includes an X-ray imaging system 110, and one or more processors 120. The X-ray imaging system 110 is configured to:
acquire S110 volumetric X-ray data 130 representing a distribution of a contrast agent injection in a region of interest in the brain; and
acquire S120 projection X-ray data 140 representing a distribution of the same contrast agent injection in the aortic arch.

The one or more processors 120 are configured to:
generate S130, from the volumetric X-ray data 130, a first image 150 for assessing the region of interest in the brain; and
generate S140, from the projection X-ray data 140, a second image 160 for planning an intravascular procedure via the aortic arch.

The above-described system 100 provides a first image 150 for assessing the region of interest in the brain, and also a second image 160 for planning an intravascular procedure via the aortic arch. Since the images 150, 160, represent distributions of the same contrast agent injection into a subject, the system provides the second image for planning the intravascular procedure via the aortic arch, without the need for an additional contrast agent injection. This avoids the unnecessary complications, and also the unnecessary contrast agent dose, associated with an additional contrast agent injection. Consequently, it may help to avoid unnecessary delays in performing the intravascular procedure.

As mentioned above, the system 100 for planning an intravascular procedure includes an X-ray imaging system 110, and one or more processors 120. The X-ray imaging system 110 is configured to acquire S110 volumetric X-ray data 130 representing a distribution of a contrast agent injection in a region of interest in the brain, and to acquire S120 projection X-ray data 140 representing a distribution of the same contrast agent injection in the aortic arch.

The X-ray imaging system 110 may be provided by various arrangements of X-ray sources, and X-ray detectors, i.e. various X-ray source-detector arrangements. These include, for example, conventional (cone beam) X-ray imaging systems, and also spectral (cone beam) X-ray imaging systems. Conventional X-ray imaging systems generate X-ray attenuation data representing X-ray attenuation within a single energy interval. By contrast, spectral X-ray imaging systems generate X-ray attenuation data representing X-ray attenuation within multiple different energy intervals. The X-ray attenuation data generated by a spectral X-ray imaging system may be processed, e.g. using various material decomposition algorithms, in order to distinguish between media that have similar X-ray attenuation values when measured within a single energy interval, and which would be indistinguishable in X-ray attenuation data obtained using a conventional X-ray imaging system. Thus, the X-ray attenuation data generated by spectral X-ray imaging systems may be processed to provide images with improved specificity to materials such as contrast agent, tissue, bone, and so forth.

In one example the X-ray imaging system 110 includes a first X-ray source-detector arrangement 110a configured to acquire the volumetric X-ray data 130, and a second X-ray source-detector arrangement 110b configured to acquire the projection X-ray data 140. This example is illustrated in Fig. 1, and wherein the first X-ray source-detector arrangement 110a includes an X-ray source 110^{S}a and a corresponding X-ray detector 110^{D}a that are mounted to a C-arm. The second X-ray source-detector arrangement 110b includes an X-ray source 110^{S}b and a corresponding X-ray detector 110^{D}b that are likewise mounted to a C-arm. This arrangement may be provided by the Azurion 7 B20/15 Image Guided Therapy System Biplane that is marketed by Philips Healthcare, Best, The Netherlands.

With continued reference to the example illustrated in Fig. 1, the first X-ray source-detector arrangement 110a is configured to acquire the volumetric X-ray data 130 from each of a plurality of rotational angles θ of the first X-ray source-detector arrangement with respect to the region of interest in the brain. The second X-ray source-detector arrangement 110b is configured to acquire the projection X-ray data 140 from each of one or more orientations α of the second X-ray source-detector arrangement with respect to the aortic arch.

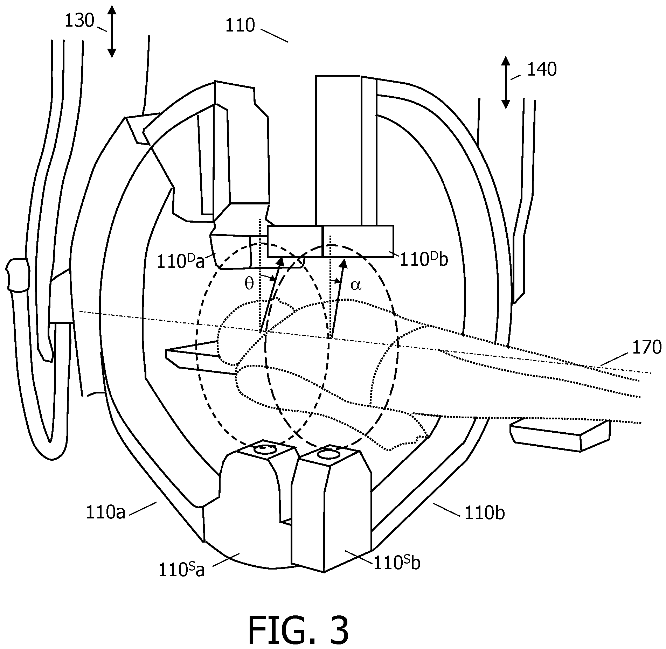

An example illustrating the positioning of the first and second X-ray source-detector arrangements 110a, 110b, with respect to a subject in order to acquire the respective volumetric X-ray data 130, and the projection X-ray data 140, is illustrated in Fig. 3. Fig. 3 is a schematic diagram illustrating the acquisition of volumetric X-ray data 130 by a first X-ray source-detector arrangement 110a from a rotational angle θ with respect to a region of interest in the brain, and the acquisition of projection X-ray data 140 by a second X-ray source-detector arrangement 110b from an orientation α with respect to the aortic arch, in accordance with some aspects of the present disclosure.

With reference to Fig. 3, in-use, the first X-ray source-detector arrangement 110a is positioned such that a field of view of the first X-ray source-detector arrangement captures the region of interest in the brain. This positioning operation may be performed manually by an operator, e.g. by acquiring initial X-ray data representing the region of interest in the brain, and then iteratively re-positioning the subject and/or the first X-ray source-detector arrangement 110a until the region of interest in the brain is captured within its field of view. This positioning operation may alternatively be performed automatically following the acquisition of such initial X-ray data, e.g. by detecting anatomical landmark(s) in the initial X-ray data, as described in an example below.

With continued reference to Fig. 3, in-use, the second X-ray source-detector arrangement 110b is positioned such that a field of view of the second X-ray source-detector arrangement captures the aortic arch. This positioning operation may be performed manually by an operator, e.g. by acquiring initial projection X-ray data representing the aortic arch, and then iteratively re-positioning either the subject or the second X-ray source-detector arrangement 110b until the aortic arch is captured within its field of view. This positioning operation may alternatively be performed automatically following the acquisition of such initial projection X-ray data, e.g. by detecting anatomical landmark(s) in the initial projection X-ray data, as described in an example below.

The positioning operations described above may include manually, or automatically, determining a position of at least one of the first X-ray source-detector arrangement 110a and the second X-ray source-detector arrangement 110b for acquiring the volumetric X-ray data 130 and the projection X-ray data 140 without the first and second X-ray source-detector arrangements obstructing one another. Some of the factors that may be taken into account in meeting this constraint are described in the examples below.

With continued reference to Fig. 1 and Fig. 2, the X-ray imaging system 110 is configured to:
acquire S110 volumetric X-ray data 130 representing a distribution of a contrast agent injection in a region of interest in the brain; and
acquire S120 projection X-ray data 140 representing a distribution of the same contrast agent injection in the aortic arch.

With reference to Fig. 3, in the operation S110, the volumetric X-ray data 130 may be acquired by rotating the first X-ray source-detector arrangement 110a around the region of interest in the brain and acquiring the volumetric X-ray data from each of a plurality of the rotational angles θ. In the example illustrated in Fig. 3, the first X-ray source-detector arrangement 110a is rotated around an axis of rotation 170 of the first X-ray source-detector arrangement 110a. The acquired X-ray data 130 is referred to as volumetric X-ray data 140 in the sense that whilst the first X-ray source-detector arrangement 110a acquires projection X-ray data representing a projection of the anatomy at each rotational angle θ, this projection X-ray data may be reconstructed using known image reconstruction techniques to provide a volumetric image of the anatomy. The acquired volumetric X-ray data 140 may also be referred-to as tomographic X-ray data, or simply as CT data. The volumetric X-ray data 130 may be acquired during a complete revolution of the first X-ray source-detector arrangement 110a around the region of interest in the brain. Thus, the range of rotational angles θ over which the volumetric X-ray data 130 is acquired may be greater than or equal to 360 degrees. Alternatively, the volumetric X-ray data 130 may be acquired during a partial revolution of the first X-ray source-detector arrangement 110a around the region of interest in the brain. Thus, the range of rotational angles θ over which the volumetric X-ray data 130 is acquired may be less than 360 degrees.

With reference to Fig. 3, in the operation S120, the projection X-ray data 140 may be acquired by positioning the second X-ray source-detector arrangement 110b at each of one or more orientations α with respect to the aortic arch. The acquired X-ray data 140 is referred to as projection X-ray data 130 in the sense that the second X-ray source-detector arrangement 110b acquires projection X-ray data representing a projection of the anatomy at each orientation α. In one example, the projection X-ray data 140 is acquired by positioning the second X-ray source-detector arrangement 110b at a single orientation α with respect to the aortic arch. This projection X-ray data may be used to generate a projection image of the aortic arch. In another example, the projection X-ray data 140 is acquired by positioning the second X-ray source-detector arrangement 110b at each of a plurality of orientations α with respect to the aortic arch. The second X-ray source-detector arrangement 110b may be positioned at each of two or more orientations α with respect to the aortic arch, for example. In this example, the projection X-ray data that is acquired from the multiple orientations α with respect to the aortic arch may be reconstructed using known image reconstruction techniques to provide a volumetric image of the aortic arch. In this example, the projection X-ray data 140 may be acquired during a partial, or a complete revolution of the second X-ray source-detector arrangement 110b around the aortic arch. Thus, the range of orientations α over which the projection X-ray data 140 is acquired may be less than 360 degrees, or it may be greater than or equal to 360 degrees.

In the example illustrated in Fig. 1, the first and second X-ray source-detector arrangements 110a, 110b, may be referred-to as projection X-ray imaging systems. Volumetric angiographic images that are obtained from projection X-ray imaging systems such as those described above are typically referred-to as a 3D rotational angiograms "3DRA"s. However, it is noted that the first and second X-ray source-detector arrangements 110a, 110b, may alternatively be provided in a different manner to that illustrated in Fig. 1. For instance, in one example, the first X-ray source-detector arrangement 110a is provided by a (cone beam) CT imaging system. In another example, instead of using the C-arms illustrated in Fig. 1, the use of other types of supports for the X-ray sources 110^{S}a,b and X-ray detectors 110^{D}a,b are contemplated. In yet another example, the first X-ray source-detector arrangement 110a and the second X-ray source-detector arrangement 110b share a common X-ray source. In this example, the one or more processors 120 selectively direct X-ray radiation emitted by the common X-ray source to an X-ray detector of the first X-ray source-detector arrangement 110a for acquiring the volumetric X-ray data 130, or to an X-ray detector of the second X-ray source-detector arrangement 110b for acquiring the projection X-ray data 140.

With reference to Fig. 2, the volumetric X-ray data 130 and the projection X-ray data 140 that are acquired in the operations S110 and S120, represent distributions of the same contrast agent injection into the subject. In other words, the volumetric X-ray data 130 and the projection X-ray data 140 are acquired subsequent to the same contrast agent injection into a subject. The contrast agent provides visibility of the vasculature into which the contrast agent is injected. The contrast agent may be injected manually or automatically, e.g. via an injector. The contrast agent may be injected using the injector 180 illustrated in Fig. 1, for example. The injector 180 may be controlled by the one or more processors 120, as illustrated in Fig. 1. The contrast agent may be injected into the subject via the leg, or via the hand, or via another location in the anatomy. The contrast agent may be injected in the form of a bolus, for example. The contrast agent may include a substance such as Iodine, or a Lanthanide such as Gadolinium, or another substance that provides visibility of the vasculature into which the contrast agent is injected.

Various protocols for acquiring the volumetric X-ray data 130 and the projection X-ray data 140, are contemplated. These include acquiring the volumetric X-ray data 130 and the projection X-ray data 140 simultaneously, or during overlapping periods, or consecutively. In some examples, the timing of the acquisition of these data depends on factors such as the location in the anatomy into which the contrast agent is injected. For instance if the contrast agent is injected into a venous region, e.g. in the hand or the leg, the contrast agent will reach the aortic arch prior to reaching the brain. In this situation, the acquisition of the projection X-ray data 140 representing a distribution of a contrast agent injection in the aortic arch may begin prior to the acquisition of the volumetric X-ray data 130 representing the distribution of the same contrast agent injection in a region of interest in the brain. In some cases, the acquisition of the projection X-ray data 140 may terminate prior to beginning the acquisition of the volumetric X-ray data 130. In other cases, the acquisition of the projection X-ray data 140 may overlap in time with the acquisition of the volumetric X-ray data 130. In other situations, the acquisition of the projection X-ray data 140 representing a distribution of a contrast agent injection in the aortic arch may begin simultaneously with the acquisition of the volumetric X-ray data 130 representing the distribution of the same contrast agent in a region of interest in the brain.

Referring now to the operations S130, and S140 illustrated in Fig. 2, in these operations, the one or more processors 120 are configured to:
generate S130, from the volumetric X-ray data 130, a first image 150 for assessing the region of interest in the brain; and
generate S140, from the projection X-ray data 140, a second image 160 for planning an intravascular procedure via the aortic arch.

Thus, the volumetric X-ray data 130, and the projection X-ray data 140, described above, may be received by the one or more processors 120. These data may be received by the one or more processors 120 via any form of data communication. For instance, these data may be received via wired, or wireless, or optical fiber communication, for example. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

The first image 150 that is generated in the operation S130 may be used to assess the region of interest in the brain. This assessment may be performed in various ways, such as manually by a physician, or automatically by an image analysis algorithm. The assessment may include diagnosing the region of interest to determine a location and a severity of a stenosis in the vasculature of the brain, for example. The first image 150 for assessing the region of interest in the brain may be a volumetric image, or a projection image. In the former case, a volumetric image may be generated by reconstructing the volumetric X-ray data 130 using known image reconstruction techniques. In the latter case, projection image may be generated from the volumetric image, e.g. as an image slice. Alternatively, a projection image may be generated directly from the volumetric X-ray data 130 using X-ray data that has been acquired from one of the rotational angles θ of the first X-ray source-detector arrangement with respect to the region of interest in the brain.

The second image 160 that is generated in the operation S 140 may be used to plan an intravascular procedure via the aortic arch. This planning operation may be performed in various ways, such as manually by a physician, or automatically by an image analysis algorithm. The intravascular procedure may be an intravascular thrombectomy procedure for the treatment of a suspected occlusion in the brain, for example. The intravascular procedure may alternatively be another procedure, such as such as a thrombolysis procedure, an angioplasty procedure, a stenting procedure, a coiling procedure, and so forth. Such procedures may involve the navigation of various interventional devices (e.g. guidewire(s), catheter(s), and treatment devices) to the location of the suspected occlusion in the brain, via the aortic arch.

In one example, the projection X-ray data 140 representing a distribution of a contrast agent injection in the aortic arch is acquired from a single orientation α of the second X-ray source-detector arrangement with respect to the aortic arch. In this example, the second image 160 for planning the intravascular procedure via the aortic arch, and which is generated from the projection X-ray data 140, may be a projection image. In another example, the projection X-ray data 140 representing a distribution of a contrast agent injection in the aortic arch is acquired from multiple orientations α of the second X-ray source-detector arrangement with respect to the aortic arch. In this example, the second image 160 for planning the intravascular procedure via the aortic arch, and which is generated from the projection X-ray data 140, may be a volumetric image. The volumetric image may be reconstructed from the projection X-ray data 140 using known image reconstruction techniques.

The first and second images 150, 160, may then be outputted by the one or more processors. The first and second images 150, 160, may be outputted in various ways. For example these images may be outputted to a display device, such as to the display 190 illustrated in Fig. 1, or to a virtual/ augmented reality display device, or to a printer, or to a computer-readable storage medium, or to the Internet, or to the Cloud, and so forth.

As mentioned above, since the images 150, 160 represent distributions of the same contrast agent injection into a subject, the system provides the second image 160 for planning the intravascular procedure via the aortic arch, without the need for an additional contrast agent injection. This avoids the unnecessary complications, and also the unnecessary contrast agent dose, associated with an additional contrast agent injection. Consequently, it may help to avoid unnecessary delays in performing the intravascular procedure.

One or more additional operations may be performed by the one or more processors 120 of the system 100 illustrated in Fig. 1, as described in the examples below.

In one example, the one or more processors 120 are configured to determine a position of at least one of the first X-ray source-detector arrangement 110a and the second X-ray source-detector arrangement 110b for acquiring the volumetric X-ray data 130 and the projection X-ray data 140 without the first and second X-ray source-detector arrangements obstructing one another.

Various factors may be taken into consideration in this example, as described in the example implementations below. It is noted that these example implementations may be implemented alone, or as a combination of two or more of the example implementations.

In some of the example implementations described below, a current position of the first and/or second X-ray source-detector arrangement(s) is used to determine the position of the first and/or second X-ray source-detector arrangements 110a, 110b, for acquiring the volumetric X-ray data 130 and the projection X-ray data 140 without the first and second X-ray source-detector arrangements obstructing one another. The current positions of the X-ray source-detector arrangements 110, 110b, may be determined in various ways. For instance, they may be determined from position data that is acquired from various sensors (e.g. linear encoders, rotary encoders, and so forth) that are used in positioning the X-ray source-detector arrangement(s) 110, 110b. The position data may alternatively be determined from video data generated by one or more cameras that capture the X-ray source-detector arrangement(s) 110a, 110b. For instance, the current position of the X-ray source-detector arrangement(s) 110a, 110b, may be determined by performing object recognition techniques on the video data. The current positions of the first and/or second X-ray source-detector arrangement(s) may be used in combination with a mathematical model describing the spatial freedom of movement of the first and/or X-ray source-detector arrangement(s) 110, 110b to determine the position(s) of the first and/or second X-ray source-detector arrangements 110a, 110b, for acquiring the volumetric X-ray data 130 and the projection X-ray data 140 without the first and second X-ray source-detector arrangements obstructing one another. The model may for example, be used to identify, and therefore avoid, position conflicts in which the positions of elements of the first and second X-ray source-detector arrangements (e.g. the X-ray source, the X-ray detector, the C-arm, and so forth) coincide in space and/or time.

In one example implementation, the operation of determining a position of at least one of the first X-ray source-detector arrangement 110a and the second X-ray source-detector arrangement 110b comprises: determining a position of the first X-ray source-detector arrangement 110a such that a field of view of the first X-ray source-detector arrangement captures the region of interest in the brain.

In this example implementation, the position of the first X-ray source-detector arrangement 110a may be determined based on the above-described current position of the first X-ray source-detector arrangement 110a, and based on the above-described mathematical model describing the spatial freedom of movement of the first X-ray source-detector arrangement 110a. The mathematical model may further describe the field of view of the first X-ray source-detector arrangement 110a in relation to its current position. This model may be used in combination with a further mathematical model describing the current position of the subject's brain in relation to the current position of the first X-ray source-detector arrangement 110a. This further mathematical model may, for example, provide an estimate the current position of the subject's brain in relation to the current position of the first X-ray source-detector arrangement 110a, based on a current position of a patient bed, and an anatomical model of the subject's brain.

In another example implementation, the operation of determining a position of at least one of the first X-ray source-detector arrangement 110a and the second X-ray source-detector arrangement 110b comprises: determining a position of the second X-ray source-detector arrangement 110b such that a field of view of the second X-ray source-detector arrangement captures the aortic arch.

In this example implementation, the position of the second X-ray source-detector arrangement 110b may be determined based on the above-described current position of the second X-ray source-detector arrangement 110b, and based on the above-described mathematical model describing the spatial freedom of movement of the second X-ray source-detector arrangement 110b. The mathematical model may further describe the field of view of the second X-ray source-detector arrangement 110b in relation to its current position. This model may be used in combination with a further mathematical model describing the current position of the subject's aortic arch in relation to the current position of the second X-ray source-detector arrangement 110b. This further mathematical model may, for example, provide an estimate the current position of the subject's aortic arch in relation to the current position of the second X-ray source-detector arrangement 110b, based on a current position of a patient bed, and an anatomical model of the subject's aortic arch.

In another example implementation, the operation of determining a position of at least one of the first X-ray source-detector arrangement 110a and the second X-ray source-detector arrangement 110b comprises: determining a position of the second X-ray source-detector arrangement 110b to provide approximately an anterior-posterior, AP, view of the aortic arch.

In this example implementation, the position of the second X-ray source-detector arrangement 110b may be determined as described above in the previous example implementation, the AP view being derived from the anatomical model of the subject's aortic arch.

In another example implementation, the operation of determining a position of at least one of the first X-ray source-detector arrangement 110a and the second X-ray source-detector arrangement 110b comprises: determining a position of the second X-ray source-detector arrangement 110b such that the second X-ray source-detector arrangement does not obstruct a rotational path of the first X-ray source-detector arrangement 110a during a rotation of the first X-ray source-detector arrangement around the region of interest in the brain.

In this example implementation, the position(s) of the first and second X-ray source-detector arrangement(s) 110a, 110b, may be determined from their respective current positions, and from mathematical models describing the spatial freedom of movement of the first and/or X-ray source-detector arrangement(s) 110, 110b. The mathematical models may further describe the rotational path of the first X-ray source-detector arrangement 110a. This path may be modelled as having a circular shape, or another shape, as described below.

In another example implementation, the operation of determining a position of at least one of the first X-ray source-detector arrangement 110a and the second X-ray source-detector arrangement 110b comprises: determining a position of the second X-ray source-detector arrangement 110b such that the second X-ray source-detector arrangement is tilted with respect to a plane perpendicular to the axis of rotation 170 of the first X-ray source-detector arrangement 110a.

In this example implementation, a central beam between the X-ray source and the corresponding X-ray detector of the second X-ray source-detector arrangement is tilted with respect to a plane perpendicular to the axis of rotation 170 of the first X-ray source-detector arrangement 110a. Tilting the second X-ray source-detector arrangement in this manner may improve ability to visualize the region of interest in the brain and/or the aortic arch, within the field of view of the respective X-ray source-detector arrangement. As seen in Fig. 3, this is hampered by the proximity of the brain and the aortic arch, and also by the sizes of the X-ray sources 110^{S}a, 110^{S}b.

In another example implementation, the operation of determining a position of at least one of the first X-ray source-detector arrangement 110a and the second X-ray source-detector arrangement 110b comprises:
acquiring, with the first X-ray source-detector arrangement 110a, initial X-ray data representing the region of interest in the brain and/or acquiring, with the second X-ray source-detector arrangement 110b, initial projection X-ray data representing the aortic arch; and determining the position of at least one of the first X-ray source-detector arrangement and the second X-ray source-detector arrangement, based on the initial X-ray data and/or the initial projection X-ray data, respectively.

In this example implementation, the initial X-ray data representing the region of interest in the brain may be projection X-ray data, or it may be volumetric X-ray data. The initial X-ray data representing the region of interest in the brain may be projection X-ray data representing a live sequence of images of the region of interest in the brain, for example. The initial X-ray data representing the region of interest in the brain, and the initial projection X-ray data representing the aortic arch, may be used to generate corresponding images that are then used to determine the position of the first and/or second X-ray source-detector arrangement. The initial X-ray data may be used to generate a projection image or a volumetric image depending on whether projection X-ray data, or volumetric X-ray data is acquired. The initial projection X-ray data may be used to generate a projection image.

In some situations, the region of interest in the brain, and the aortic arch, may be at least weakly visible in the images that are generated from the initial X-ray data, and the initial projection X-ray data. In such cases their positions may be identified in these images using various image processing techniques, e.g. object recognition techniques. In other situations, however, the region of interest in the brain, and the aortic arch, may be weakly visible, or completely invisible in the corresponding images. Such situations arise when the initial X-ray data and initial projection X-ray data are acquired in the absence of contrast agent. In such situations, the positions of the region of interest, and likewise, the position of the aortic arch, may be estimated in the corresponding images based on the position of one or more anatomical landmark(s), such as bone landmarks from the skull, ribs, and so forth. For instance, a previously-acquired image that includes the region of interest in the brain, or the aortic arch, or an atlas image, may be registered to the respective image in order to localize the region of interest in the brain, and the aortic arch, respectively. The image generated from the initial X-ray data (representing the region of interest in the brain) may then be used to determine the position of the first X-ray source-detector arrangement 110a with respect to the region of interest in the brain, and consequently to adjust its position such that a field of view of the first X-ray source-detector arrangement captures the region of interest in the brain. The image generated from the initial projection X-ray data (representing the aortic arch) may be used to determine the position of the second X-ray source-detector arrangement 110b with respect to the aortic arch, and consequently to adjust its position such that such that a field of view of the second X-ray source-detector arrangement captures the aortic arch.

In another example implementation, the operation of determining a position of at least one of the first X-ray source-detector arrangement 110a and the second X-ray source-detector arrangement 110b comprises:
determining an initial position of the second X-ray source-detector arrangement 110b for acquiring the projection X-ray data 140 and wherein the second X-ray source-detector arrangement obstructs a rotational path of the first X-ray source-detector arrangement 110a during a rotation of the first X-ray source-detector arrangement around the region of interest in the brain, and determining a subsequent position of the second X-ray source-detector arrangement 110b wherein the second X-ray source-detector arrangement does not obstruct the rotational path of the first X-ray source-detector arrangement 110a during a rotation of the first X-ray source-detector arrangement around the region of interest in the brain; and wherein the one or more processors 120 are configured to adjust the position of the second X-ray source-detector arrangement 110b between the initial position and the subsequent position after acquiring the projection X-ray data and before the acquiring of the volumetric X-ray data is completed.

In this example implementation, the adjusting of the position of the second X-ray source-detector arrangement 110b between the initial position and the subsequent position may enable the acquisition of improved images region of interest in the brain and/or the aortic arch. In one example, the adjusting of the position of the second X-ray source-detector arrangement 110b between the initial position and the subsequent position, occurs prior to, or simultaneously with, the start of the rotation of the first X-ray source-detector arrangement around the region of interest in the brain, i.e. prior to, or simultaneously with, the start of the acquisition of the volumetric X-ray data (130). In another example, the adjusting of the position of the second X-ray source-detector arrangement 110b between the initial position and the subsequent position, occurs during the rotation of the first X-ray source-detector arrangement around the region of interest in the brain. For instance, in one example, the adjusting of the position of the second X-ray source-detector arrangement 110b between the initial position and the subsequent position, occurs based on the acquisition of sufficient projection X-ray data 140 to generate a second image that represents the aortic arch with a predetermined image quality.

In this example implementation, the initial and subsequent positions of the second X-ray source-detector arrangement 110b, may be determined based on the current positions of the first and second X-ray source-detector arrangement(s) 110a, 110b, and based on the above-described mathematical models describing the spatial freedom of movement of the first and X-ray source-detector arrangements 110, 110b, and also based on the above-described mathematical models describing the current position of the subject's brain and aortic arch in relation to the current position of the first and second X-ray source-detector arrangements 110a, 110b.

In another example implementation, the operation of determining a position of at least one of the first X-ray source-detector arrangement 110a and the second X-ray source-detector arrangement 110b comprises:
dynamically adjusting a path of the first X-ray source-detector arrangement 110a during a rotation of the first X-ray source-detector arrangement around the region of interest in the brain such that the path of the first X-ray source-detector arrangement deviates from a circular trajectory whilst acquiring the volumetric X-ray data 130 and such that the second X-ray source-detector arrangement 110b does not obstruct the path of the first X-ray source-detector arrangement.

In this example implementation, the path of the first X-ray source-detector arrangement deviates from a circular trajectory. The path may for example describe a butterfly-shaped trajectory, or a trajectory with another shape. By deviating from a circular trajectory, the source-detector arrangement(s) may be positioned more optimally with respect to the region of interest in the brain and/or the aortic arch, facilitating improved images of these regions to be acquired.

In one example, the one or more processors 120 are configured to generate control signals for controlling one or more actuators to automatically position the at least one of the first X-ray source-detector arrangement 110a and the second X-ray source-detector arrangement 110b in the determined position.

The automatic positioning that is provided in accordance with this example may help to avoid delays in obtaining the first and second images 150, 160.

In one example, a value of one or more image acquisition parameters of the second X-ray source-detector arrangement 110b is adjusted based on a position of a front of the contrast agent injection in relation to the aortic arch. In this example, the second X-ray source-detector arrangement 110b is configured to acquire projection X-ray data representing a position of a front of the contrast agent injection in relation to the aortic arch. The one or more processors 120 are configured to:
automatically adjust a value of one or more image acquisition parameters of the second X-ray source-detector arrangement 110b between a first set of one or more parameter values for acquiring the projection X-ray data representing the position of the front of the contrast agent injection in relation to the aortic arch, and a second set of one or more parameter values for acquiring the projection X-ray data 140 representing the distribution of the contrast agent injection in the aortic arch; and
wherein the automatic adjusting, is performed based on a position of the front of the contrast agent injection in relation to the aortic arch determined from the projection X-ray data representing the position of the front of the contrast agent injection in relation to the aortic arch.

In this example, the automatic adjusting may be triggered based on the position of the front of the contrast agent injection approaching, or reaching, or partially filling, or completely filling, the aortic arch, for example. The image acquisition parameters that are adjusted may include one or more of: a frame rate, an X-ray dose, and an energy of the X-ray radiation, for example. By way of an example, relatively lower values of the frame rate and/or X-ray dose and/or X-ray energy may be used as the first set of parameter value(s), and relatively higher values of the frame rate and/or X-ray dose and/or X-ray energy may be used as the second set of parameter value(s). This may facilitate the acquisition of the second image 160 for planning an intravascular procedure via the aortic arch, at an optimal time, or with improved image quality, or with reduced X-ray dose to the subject. In this example, the one or more processors 120 may also generate, and output, a recommendation for a type of interventional device to be used to pass the aortic arch. Such recommendations may be generated from a look-up table, for example.

In one example, the second X-ray source-detector arrangement 110b is configured to acquire projection X-ray data representing a position of a front of the contrast agent injection in relation to the aortic arch. In this example, the one or more processors 120 are configured to:
automatically trigger the acquiring of the volumetric X-ray data 130 representing the distribution of a contrast agent injection in the region of interest in the brain, based on the position of the front of the contrast agent injection in relation to the aortic arch.

In this example, the triggering of the acquiring of the volumetric X-ray data 130 may occur based on the position of the front of the contrast agent injection (as detected in the projection X-ray data acquired by the second X-ray source-detector arrangement 110b) approaching, or reaching, or partially filling, or completely filling, the aortic arch, for example. The triggering of the acquiring of the volumetric X-ray data 130 may alternatively occur subsequent to a predetermined delay following such events. For example, it may be known that there is typically a predetermined delay between the front of the contrast agent injection reaching the aortic arch, and the contrast agent reaching the brain. In both cases, the position of the front of the contrast agent injection in relation to the aortic arch, is used to trigger the acquisition of the volumetric X-ray data 130. The triggering of the acquisition of the volumetric X-ray data 130 may include initiating a rotation of the first X-ray source-detector arrangement, for example.

Alternatively or additionally, in this example, the one or more processors 120 are configured to:
automatically trigger an adjustment to a position of the second X-ray source-detector arrangement 110b such that the second X-ray source-detector arrangement does not obstruct a rotational path of the first X-ray source-detector arrangement during a rotation of the first X-ray source-detector arrangement around the region of interest in the brain, based on the position of the front of the contrast agent injection in relation to the aortic arch.

In this example, the position of the second X-ray source-detector arrangement 110b may be adjusted so that its source, or its detector, or its support arm, does not obstruct the rotational path of the first X-ray source-detector arrangement, for example. The automatic triggering in this example may occur based on the acquisition of sufficient projection X-ray data 140 to generate a second image that represents the aortic arch with a predetermined image quality. This may facilitate the generation of an improved second image 160 for planning the intravascular procedure via the aortic arch, whilst at the same time avoiding that the second X-ray source-detector arrangement 110b obstructs the rotational path of the first X-ray source-detector arrangement.

In one example, the one or more processors 120 are configured to: calculate, based on the volumetric X-ray data 130 and the projection X-ray data 140, a time delay between the contrast agent injection reaching the aortic arch and the contrast agent injection reaching the brain; and output an indication of the time delay.

In this example, the time delay may be generated using timestamps indicating the times at which the volumetric X-ray data 130 and the projection X-ray data 140, are acquired. The time delay gives an indication of the overall speed of the blood flow to the brain, and may be used by a physician in assessing the region of interest in the brain, e.g. in assessing the severity of a suspected occlusion.

In one example, the one or more processors 120 are configured to:
acquire projection X-ray data representing an intravascular device in the aortic arch during the intravascular procedure;
generate, from the projection X-ray data, a third image representing the intravascular device in the aortic arch; and
output, to a display device, the second image and the third image for assisting a navigation of the intravascular device along the aortic arch during the intravascular procedure.

In this example, the second and third images may be outputted to a display device such as the display 190 illustrated in Fig. 1, or to a virtual/ augmented reality display device, or to a printer, or to a computer-readable storage medium, or to the Internet, or to the Cloud, and so forth. The projection X-ray data representing the intravascular device in the aortic arch during the intravascular procedure may be acquired periodically, and in real-time. Thus, the third image may be a live image of the intravascular device in the aortic arch during the intravascular procedure. The second and third images may be outputted in various ways to the display device, such as side-by-side, or as an overlay image. The overlay image may be generated by mutually-registering the images. Since the second and third images are both displayed, the physician may visualize the shape of the aortic arch (via the second image) as well as the interventional device in the aortic arch (via the third image), which assists the physician in navigating the interventional device past the aortic arch. In this example, the intravascular device may be a guidewire, or a catheter, a treatment device, or another type of intravascular device.

In a related example, the one or more processors 120 are configured to: generate control signals for controlling one or more actuators to automatically position the first X-ray source-detector arrangement 110a or the second X-ray source-detector arrangement 110b to acquire the projection X-ray data representing the intravascular device in the aortic arch during the intravascular procedure from a same orientation as the orientation used to acquire the projection X-ray data 140 used to generate the second image 160.

In this example, the projection X-ray data representing the intravascular device in the aortic arch during the intravascular procedure may be acquired by the first or the second X-ray source-detector arrangement 110b. For instance, in the system 100 illustrated in Fig. 1, the projection X-ray data representing the intravascular device in the aortic arch during the intravascular procedure may be acquired by the first X-ray source-detector arrangement 110a, or the second X-ray source-detector arrangement 110b. The use of the same orientation to acquire the projection X-ray data representing the intravascular device in the aortic arch during the intravascular procedure, as the orientation used to acquire the projection X-ray data 140 used to generate the second image 160, ensures that the second and third images provide the same perspectives of the aortic arch. This facilitates improved navigation of the interventional device past the aortic arch. The same orientation, in this context, is intended to refer to orientations that are within approximately ±5 degrees of one another.

It is noted that in addition to the X-ray imaging system 110 and the one or more processors 120, the system 100 described above may also include one or more of: a display device (such as the display 190 illustrated in Fig. 1) for displaying the first image 150, the second image 160, and the third image, other outputs generated by the one or more processors 120, and so forth; an injector 180 for providing the contrast agent injection into the subject; a patient bed; and a user input device (not illustrated in Fig. 1) configured to receive user input in relation to the operations performed by the one or more processors 120, such as a keyboard, a mouse, a touchscreen, and so forth.

In another example, a computer-implemented method of planning an intravascular procedure, is provided. The method includes:
causing an X-ray imaging system 110 to acquire S110 volumetric X-ray data 130 representing a distribution of a contrast agent injection in a region of interest in the brain; and
causing an X-ray imaging system 110 to acquire S120 projection X-ray data 140 representing a distribution of the same contrast agent injection in the aortic arch;
generating S130, from the volumetric X-ray data 130, a first image 150 for assessing the region of interest in the brain; and
generating S140, from the projection X-ray data 140, a second image 160 for planning an intravascular procedure via the aortic arch.

In another example, a computer program product is provided. The computer program product comprises instructions which when executed by one or more processors 120, cause the one or more processors to carry out a method comprising:
causing an X-ray imaging system 110 to acquire S110 volumetric X-ray data 130 representing a distribution of a contrast agent injection in a region of interest in the brain; and
causing an X-ray imaging system 110 to acquire S120 projection X-ray data 140 representing a distribution of the same contrast agent injection in the aortic arch;
generating S130, from the volumetric X-ray data 130, a first image 150 for assessing the region of interest in the brain; and
generating S140, from the projection X-ray data 140, a second image 160 for planning an intravascular procedure via the aortic arch.

The above examples are to be understood as illustrative of the present disclosure, and not restrictive. Further examples are also contemplated. For instance, the examples described in relation to a system, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for planning an intravascular procedure, the system comprising:
an X-ray imaging system (110); and
one or more processors (120);
wherein the X-ray imaging system (110) is configured to:
acquire (S110) volumetric X-ray data (130) representing a distribution of a contrast agent injection in a region of interest in the brain; and
acquire (S120) projection X-ray data (140) representing a distribution of the same contrast agent injection in the aortic arch; and
wherein the one or more processors (120) are configured to:
generate (S130), from the volumetric X-ray data (130), a first image (150) for assessing the region of interest in the brain; and
generate (S140), from the projection X-ray data (140), a second image (160) for planning an intravascular procedure via the aortic arch.

2. The system according to claim 1, wherein the X-ray imaging system (110) comprises a first X-ray source-detector arrangement (110a) configured to acquire the volumetric X-ray data (130), and a second X-ray source-detector arrangement (110b) configured to acquire the projection X-ray data (140).

3. The system according to claim 2, wherein the first X-ray source-detector arrangement (110a) is configured to acquire the volumetric X-ray data (130) from each of a plurality of rotational angles (θ) of the first X-ray source-detector arrangement with respect to the region of interest in the brain; and
wherein the second X-ray source-detector arrangement (110b) is configured to acquire the projection X-ray data (140) from each of one or more orientations (α) of the second X-ray source-detector arrangement with respect to the aortic arch.

4. The system according to claim 3, wherein the second X-ray source-detector arrangement (110b) is configured to acquire the projection X-ray data (140) from each a plurality of orientations (α) of the second X-ray source-detector arrangement with respect to the aortic arch; and
wherein the generating (S140) a second image (160) comprises reconstructing the projection X-ray data acquired from the plurality of orientations to provide a volumetric image for planning the intravascular procedure via the aortic arch.

5. The system according to claim 2, wherein the one or more processors (120) are further configured to determine a position of at least one of the first X-ray source-detector arrangement (110a) and the second X-ray source-detector arrangement (110b) for acquiring the volumetric X-ray data (130) and the projection X-ray data (140) without the first and second X-ray source-detector arrangements obstructing one another.

6. The system according to claim 5, wherein the determining a position of at least one of the first X-ray source-detector arrangement (110a) and the second X-ray source-detector arrangement (110b) comprises one or more of:
determining a position of the second X-ray source-detector arrangement (110b) such that the second X-ray source-detector arrangement does not obstruct a rotational path of the first X-ray source-detector arrangement (110a) during a rotation of the first X-ray source-detector arrangement around the region of interest in the brain; and
determining an initial position of the second X-ray source-detector arrangement (110b) for acquiring the projection X-ray data (140) and wherein the second X-ray source-detector arrangement obstructs a rotational path of the first X-ray source-detector arrangement (110a) during a rotation of the first X-ray source-detector arrangement around the region of interest in the brain, and determining a subsequent position of the second X-ray source-detector arrangement (110b) wherein the second X-ray source-detector arrangement does not obstruct the rotational path of the first X-ray source-detector arrangement (110a) during a rotation of the first X-ray source-detector arrangement around the region of interest in the brain; and wherein the one or more processors (120) are configured to adjust the position of the second X-ray source-detector arrangement (110b) between the initial position and the subsequent position after acquiring the projection X-ray data and before the acquiring of the volumetric X-ray data is completed.

7. The system according to claim 5 or claim 6, wherein the determining a position of at least one of the first X-ray source-detector arrangement (110a) and the second X-ray source-detector arrangement (110b) comprises one or more of:
determining a position of the first X-ray source-detector arrangement (110a) such that a field of view of the first X-ray source-detector arrangement captures the region of interest in the brain;
determining a position of the second X-ray source-detector arrangement (110b) such that a field of view of the second X-ray source-detector arrangement captures the aortic arch;
determining a position of the second X-ray source-detector arrangement (110b) to provide approximately an anterior-posterior, AP, view of the aortic arch;
determining a position of the second X-ray source-detector arrangement (110b) such that the second X-ray source-detector arrangement is tilted with respect to a plane perpendicular to the axis of rotation (170) of the first X-ray source-detector arrangement (110a);
acquiring, with the first X-ray source-detector arrangement (110a), initial X-ray data representing the region of interest in the brain and/or acquiring, with the second X-ray source-detector arrangement (110b), initial projection X-ray data representing the aortic arch; and determining the position of at least one of the first X-ray source-detector arrangement and the second X-ray source-detector arrangement, based on the initial X-ray data and/or the initial projection X-ray data, respectively;
dynamically adjusting a path of the first X-ray source-detector arrangement (110a) during a rotation of the first X-ray source-detector arrangement around the region of interest in the brain such that the path of the first X-ray source-detector arrangement deviates from a circular trajectory whilst acquiring the volumetric X-ray data (130) and such that the second X-ray source-detector arrangement (110b) does not obstruct the path of the first X-ray source-detector arrangement.

8. The system according to claim 6 or claim 7, wherein the one or more processors (120) are further configured to generate control signals for controlling one or more actuators to automatically position the at least one of the first X-ray source-detector arrangement (110a) and the second X-ray source-detector arrangement (110b) in the determined position.

9. The system according to any previous claim, wherein the second X-ray source-detector arrangement (110b) is further configured to acquire projection X-ray data representing a position of a front of the contrast agent injection in relation to the aortic arch; and
wherein the one or more processors (120) are further configured to:
automatically adjust a value of one or more image acquisition parameters of the second X-ray source-detector arrangement (110b) between a first set of one or more parameter values for acquiring the projection X-ray data representing the position of the front of the contrast agent injection in relation to the aortic arch, and a second set of one or more parameter values for acquiring the projection X-ray data (140) representing the distribution of the contrast agent injection in the aortic arch; and
wherein the automatic adjusting, is performed based on a position of the front of the contrast agent injection in relation to the aortic arch determined from the projection X-ray data representing the position of the front of the contrast agent injection in relation to the aortic arch.

10. The system according to any previous claim, wherein the second X-ray source-detector arrangement (110b) is further configured to acquire projection X-ray data representing a position of a front of the contrast agent injection in relation to the aortic arch; and
wherein the one or more processors (120) are further configured to:
automatically trigger the acquiring of the volumetric X-ray data (130) representing the distribution of the contrast agent injection in the region of interest in the brain, based on the position of the front of the contrast agent injection in relation to the aortic arch;
and/or
automatically trigger an adjustment to a position of the second X-ray source-detector arrangement (110b) such that the second X-ray source-detector arrangement does not obstruct a rotational path of the first X-ray source-detector arrangement during a rotation of the first X-ray source-detector arrangement around the region of interest in the brain, based on the position of the front of the contrast agent injection in relation to the aortic arch.

11. The system according to claim 10, wherein the one or more processors (120) are further configured to:
calculate, based on the volumetric X-ray data (130) and the projection X-ray data (140), a time delay between the contrast agent injection reaching the aortic arch and the contrast agent injection reaching the brain; and
output an indication of the time delay.

12. The system according to any previous claim, wherein the one or more processors (120) are further configured to:
acquire projection X-ray data representing an intravascular device in the aortic arch during the intravascular procedure;
generate, from the projection X-ray data, a third image representing the intravascular device in the aortic arch; and
output, to a display device, the second image and the third image for assisting a navigation of the intravascular device along the aortic arch during the intravascular procedure.

13. The system according to claim 12 when dependent on claim 2, wherein the one or more processors (120) are further configured to:
generate control signals for controlling one or more actuators to automatically position the first X-ray source-detector arrangement (110a) or the second X-ray source-detector arrangement (110b) to acquire the projection X-ray data representing the intravascular device in the aortic arch during the intravascular procedure from a same orientation as the orientation used to acquire the projection X-ray data (140) used to generate the second image (160).

14. A computer-implemented method of planning an intravascular procedure, the method comprising:
causing an X-ray imaging system (110) to acquire (S110) volumetric X-ray data (130) representing a distribution of a contrast agent injection in a region of interest in the brain; and
causing an X-ray imaging system (110) to acquire (S120) projection X-ray data (140) representing a distribution of the same contrast agent injection in the aortic arch;
generating (S130), from the volumetric X-ray data (130), a first image (150) for assessing the region of interest in the brain; and
generating (S140), from the projection X-ray data (140), a second image (160) for planning an intravascular procedure via the aortic arch.

15. A computer program product comprising instructions which when executed by one or more processors (120), cause the one or more processors to carry out the method according to claim 14.
